# EUROPEAN PATENT SPECIFICATION

(11) **EP 0 616 812 B1**
(45) Date of publication and mention of the grant of the patent: **03.11.1999**
(21) Application number: 93250090.3
(22) Date of filing: 24.03.1993
(51) Int. Cl.: A61K 39/395

(54) **Combination with anti-hormonal compounds and binding molecules for the treatment of cancer**
Kombination von Antihormonale und bindende Moleküle zur Krebsbehandlung
Combinaison d'agents anti-hormonause et molécules de liaison pour le traitement du cancer

(43) Date of publication of application: 28.09.1994
(73) Proprietor: BERLEX BIOSCIENCES, Richmond, California 94804 (US)
(72) Inventor: Langton,Beatrice C., Walnut Creek,CA 94598 (US)
(74) Representative: Fischer, Klaus Dieter

(56) References cited:
- WO-A-91/02062

## Description

The invention refers to anti-hormonal compounds which are used in combination with a further compound, like anti - c-erb B-2 antibodies (binding molecules). Additionally the invention refers to the use of the combination of the anti-hormonal compound and the second compound for the manufacture of a medicament for treatment of cancer. Further the invention refers to the process of manufacturing of the combination. Further, the invention refers to the process of administering the combination.

### State of Art

A variety of tumors have been characterised as being responsive to anti-hormonal therapies, among them breast, ovarian, and prostate. Anti-hormonal therapy has met with success in many patients, however, because anti-hormonal agents are cytostatic (i.e. they inhibit growth) rather than cytotoxic they must be given to patients for an extended period of time.

Oncogenes and their products appear to be associated with the transformation of cells to malignancy. Many of the oncogenes thus far identified have properties of cell-surface growth factor receptors. Tyrosine kinases such as the epidermal growth factor receptor (EGFr) and c-erb B-2 are two well known examples.

While many of these oncogene proteins are present on the surface of some normal cells the amplification of the oncogene or overexpression of protein product has been shown to correlate with tumorgenic phenotype. In fact, the amplification of the c-erb B2 oncogene indicates a very poor clinical prognosis, especially in breast and ovarian cancer (Slamon et al. (1987) Science **235**: 177; Slamon et al. (1989) Science **244:** 707).

The relative roles of both hormones and growth factors in the development and persistence of cancer are not well understood. The scientific literature describes conflicting results and theories. Several studies into the relationship between hormones and the putative growth factor receptor c-erb B-2 have indicated an inverse relationship between the presence of hormone receptors and the expression of c-erb B-2 in tumor cells. (See for example Beckmann et al. (1992) European Journal of Cancer **28:** 322-326; Berns et al. (1992) European Journal of Cancer **28:** 697 - 700). Mizukami et al., tested the effects of hormones and hormone antagonists on the expression of the c-erb B-2 protein in the tumor cell line MCF-7 (expressing low levels of c-erb B-2 protein) in an *in vivo* assay in mice. A progesterone antagonist (medroxyprogesterone acetate) and an oestrogen antagonist (tamoxifen) both reduced the levels of c-erb B-2 protein and decreased cellular growth rate whereas treatment with oestrogen increased the levels of c-erb B-2 and stimulated cellular growth. (Mizukami et al. (1991) Anticancer Research, **11:** 1333 - 1338). Antoniotti et al., found the opposite result. Oestrogen treatment decreased the level of c-erb B-2 protein and increased the growth rate in T47D and ZR-75-1 cells, whereas tamoxifen increased the level of protein expressed while decreasing the growth rate of the cells. (Antoniotti et al. (1992) European Journal of Cancer **28:** 318 - 321)

Several studies have investigated the role of growth factors and growth factor receptors in the development of hormone independent tumor growth. Van Agthoven et al., for example, transfected oestrogen dependent ZR-75-1 breast cancer cells with the receptor for epidermal growth factor. Prolonged treatment of these cells with tamoxifen resulted in the loss of the estrogen receptor and in the cells becoming independent of oestrogen for growth. (van Agthoven et al., (1992) Cancer Research **52:** 5082 - 5088) Valverius et al., however, were unable to render tumor cells hormone independent using the same technique. (Valverius et al. (1990) International Journal of Cancer **46:** 712 - 718)

In clinical practice, many tumors eventually become resistant to the growth suppressing actions of the anti-hormonal compound. See, for example, Piak et al. (1986) Proceedings of the National Academy of Sciences (USA) **83:** 5521; Benz et al. (1991) Proceedings of the American Association of Cancer Research **32:** 211. The use of anti-hormonal compounds for extended periods is also associated with an increased risk of developing a second type of tumor. For example, long term use of the anti-oestrogen tamoxifen is associated with an increased risk of developing endometrial carcinoma. (Gurpide, E. (1991) Journal of the National Cancer Institute **83:** 405 - 416)

### Object of the Invention

The object of the invention is to offer a combination of anti-hormonal compounds and of further compounds in order to increase the efficiency of the treatment with the anti-hormonal compounds.

### Solution of the Problem

The problem of the invention is solved by a combination comprising
a) at least one anti-hormonal compound and
b) a binding molecule or binding molecules specifically binding the protein encoded by the c-erb B-2 oncogene,
   wherein the binding molecule or molecules induce inhibition of tumor cell proliferation.

### Advantages of the Invention

This combination is surprising. Manni et al. treated cells with tamoxifen or the growth factor TGF-β. Both compounds used alone suppressed cell growth, but were not used in combination. (Manni et al. (1991) Breast Cancer Research and Treatment **20:** 43 - 52) Arteaga et al., described the sequential use of a hormonal compound and a binding molecule. They first pre-treated three different tumor cell lines with an anti-hormonal agent (tamoxifen) and then subsequently with oestrogen in the presence or absence of an antibody binding to the EGF receptor. (Arteaga et al. (1988) Molecular Endocrinology **2:** 1064 - 1069) The addition of the antibody after pre-treatment with tamoxifen did not inhibit the growth of the treated cells.

Tamoxifen is a trepenylethylene anti-oestrogen which is particularly useful in treating breast cancer. The combination of tamoxifen and cytotoxic agents such as, for example, alkylating agents has been tested clinically without showing any benefit. (See for example Fischer et al. (1986) Journal of clinical Oncology **4:** 459 - 471) Similar results were obtained when tamoxifen was combined with an agent known to reduce prolactin, a natural stimulator of breast cell growth (Bonneterre et al. (1990) Journal of Steroid Biochemistry and Molecular Biology, **37:** 977 - 981). Studies conducted in *in vitro* cell culture assays have suggested that treatment with tamoxifen may actually protect tumor cells from the treatment effects of other anti-neoplastic agents (See for example, Clarke et al. (1986) Cancer Research **46:** 6116 - 6119) The combined use of a growth factor binding molecule and a cytotoxic compound has been previously disclosed. (Aboud-Pirak et al. (1988) J. Nat. Can. Inst. **80:** 1605 - 1611; WO 89 / 06692, published 27. 3. 1989 by Genentech Inc.). Cytotoxic compounds work by poisoning all rapidly dividing cells, a mechanism distinctly different than the anti-hormonal compounds disclosed herein.

Of particular importance is the synergistic effect of the newly-described combination.

### Definition of some Expressions

### Group of anti-hormonal compounds:

The anti-hormonal compounds comprising the anti-oestrogen compounds, the group of the anti-progesterone compounds or the anti-androgen compounds. These compounds are used in the treatment of hormone dependent cancer.

### Definition of the binding molecules:

The binding molecules are specific for c-erb B-2 protein. These compounds are capable of binding to and affecting, preferably suppressing the growth of tumor cells that express the c-erb B-2 protein on their surface. These effects can be measured by the methods described in the Examples. The binding molecules can be, for example, an antibody or fragments thereof, a peptide (synthetic, natural or recombinant), a peptidomimetic compound or a peptide corresponding to the variable region of a heavy or light chain of an antibody or a peptide corresponding to a single chain comprising the heavy and the light variable region of an antibody bound by a spacer for example glycine and serine. An example of a method for synthesising peptides is found in Pike (1987) Methods in Enzymology, **146:**353.

In all cases, murine or other non-human, human or chimeric antibodies may be used. The antibodies can be prepared in a variety of ways known in the art, depending upon whether monoclonal or polyclonal antibodies are desired. Suitable methods for preparing polyclonal antibodies are described in the Handbook of Experimental Immunology (1978) 3d ed., Weir (ed.), Blackwell Scientific Publications. The process for obtaining monoclonal antibodies is described by Köhler and Milstein, (1975) Nature, **256:** 495 - 497.

Binding molecules are particularly suited for providing specificity to the c-erb B-2 protein. For example the preferred binding molecule is the chimeric monoclonal antibody BACh 250 which consists of murine variable regions and human constant regions. The manufacture of such chimeric antibodies is described in the EP-Publication 0 120 694 filed by Celltech on 23 March, 1984.

The antibody BACh 250 is made using the method described by Beidler et al. (1988) Journal of Immunology **141:** 4053. The antibody BACh 250 is manufactured by taking the murine variable region of the antibody TAb 250 which is constructed similar to the technique disclosed in the international application with the publication No.: WO 91/02062. In competitive binding assays BACh 250 is able to compete the binding of TAb 250 and is thus substantially similar to TAb 250. In addition, BACh 250 inhibits the proliferation of tumor cells that express the c-erb B-2 protein.

A variety of antigenic materials can be used for generating suitable antibodies, including transfected cells, purified proteins or fragments thereof. The antigen can be prepared by peptide synthesis or recombinant DNA means, depending upon the length of the amino acid sequences desired for the antigen. Transfected NIH3T3 cells or SKBR3 cells as well as other cells which overexpress the c-erb B-2 protein, may also be used as the antigen, either through the use of the whole cell or cell membranes.

A number of well defined techniques are available to identify antibodies with the desired specificity, such as their ability to stain tumor cells via histochemical means, to react with intact tumor cells identified by a fluorescence-activated cell sorter (FACS), or to react with the purified oncogene gene product in either a radioimmunoprecipitation (RIP) assay or in Western blot assay.

A cocktail of specific receptor binding molecules can also be used in this combination to further enhance the effect of the synergistic drug combination.

### Definition of the c-erb B-2 protein:

The c-erb B-2 protein (also referred to as c-erb B-2 protein receptor) is a 185 kDalton glycoprotein having tyrosine kinase activity and is related to, but distinct from, the epidermal growth factor receptor (EGFr). The amino acid sequence of the c-erb B-2 protein, as well as the nucleotide sequence, has been described by Coussens et al. (1985) Science **230:** 1132.
Detection for the presence of the c-erb B-2 protein may be accomplished by well known immunoassays employing antibodies specific to the c-erb B-2 protein. Such antibodies are commercially available from Chemicon International, Inc., Temecula, CA or may be developed by standard antibody procedures. It is intended herein that the c-erb B-2 protein definition will also include those proteins occurring in other host systems that are immunologically related to the human c-erb B-2 protein. For example, a related rat gene (designated neu) has been reported in Schecter et al. (1985) Science **229:** 976. Nevertheless, the antibody TAb 250 does not cross-react with neu.

### Definition of Tumor:

Tumors or cancer to be treated with the combinations of this invention are any tumors which express, or are suspected of expressing, the c-erb B-2 oncogene gene product. These tumors include, for example: breast, ovarian, bladder, prostate, and gastric cancers. Breast and ovarian cancers are most effectively treated using the combinations herein. The c-erb B-2 protein is reported to be expressed in: solid tumors by, for example, Gutman et al. (1989) International Journal of Cancer **44:**802 - 805; in human adenocarcinomas by Yokata et al. (1986) The Lancet, 5. April, p. 765; in gastric and esophageal carcinoma by Houldsworth et al. (1990) Cancer Research **50:** 6417 - 6422; in neoplastic cervix, vulva and vagina by Berchuck et al. (1990) Obstetrics and Gynecology Survey, **76:** 381; in renal cell carcinoma by Weidner et al. (1990) Cancer Research **50:** 4504; in lung adenocarcinomas By Kern et al. (1990) Cancer Research **50:** 5184 - 5191; and Scheider et al. (1986) Molecular and Cellular Biology **6:** 955 - 958 and Park et al. (1989) Cancer Research **49:** 6605; in breast and ovarian cancer by Slamon et al. (1989) Science **244:** 707; Berchuck et al. (1990) Cancer Research **50:** 4087; by Van de Vijver et al. (1987) Oncogene **1:** 423 - 430 and Bacus et al. (1990) American Journal of Pathology **137:** 103.

### Further Embodiments

A combination is preferred wherein the binding molecule or molecules are an antibody or fragments thereof. The most preferred embodiment is the chimeric monoclonal antibody BACh 250 which has been described before.

Preferably the anti-hormonal compound of the combination of the invention is an anti-oestrogen compound, ananti-progesterone or anti-androgen. Mixtures of the anti-hormonal compounds mentioned in the last sentence in addition to the binding molecules are also possible, because it is state of art that the anti-hormonal compounds of this invention show an additive or synergistic effect when they are co-administered in the absence the binding molecules. Therefore, the invention comprises a combination of a mixture of anti-hormonal compounds and binding molecules specifically binding c-erb B-2 protein wherein the binding molecules induces inhibition of tumor cell proliferation.

Such compounds are described in the publication of the EP-Publication 0 310 542 (published 05.04.1989).

Special compounds are:
(I) 11β-[(4-N,N-dimethylamino)phenyl]-17β-hydroxy-17α-propynyl-4,9(10)-estradien-3-one,
(II) 11β-[(4-N,N-dimethylamino)phenyl]-17β-hydroxy-18-methyl-17α-propynyl-4,9(10)-estradien-3-one,
(III) 11β[(4-N,N-dimethylamino)phenyl]-17aβ-hydroxy-17aαpropynyl-D-homo-4,9(10)-16-estratrien-3-one
(IV) 11β-p-methoxyphenyl-17β-hydroxy-17α-ethynyl-4,9(10)-estradien-3-one,
(V) 11β-(4-dimethylaminophenyl)-17α-hydroxy-17β-(3-hydroxypropyl)-13-methyl-4,9-gonadien-3-one;
(VI) (Z)-2-[p-(1,2-diphenyl-1-butenyl)phenoxy]-N,N-dimethylethylamine = tamoxifen,
(VII) 1-[2-[4-(6-methoxy-2-phenyl-3,4-dihydro-1-naphthyl)phenoxy]ethyl]pyrrolidine hydrochloride = nafoxidine,
(VIII) 1-[p-(2-diethylaminoethoxy)phenyl]2-(p-methoxyphenyl)-1-phenylethanol = Mer 25,
(IX) 11α-methoxy-17α-ethynyl-1,3,5(10)-estratriene-3,17β-diol;
(X) 16β-ethylestradiol,
(XI) 11-(3,17β-dihydroxy-1,3,5(10)-estratrien-7α-yl)undecanoic-acid (N-butyl-N-methyl)amide

Further anti-oestrogens are described in the European Publication EP 0 062 503 published 13 October 1982 and in M.M. GOTTARDIS et al. (1990) Cancer Research **50:** 3189 - 3192.

The group compounds with anti-androgen activity are disclosed in the US Patent with the Patent Number 5.053,405, issued 1 October 1991 in the German Publication DE 31 21 152, published 9 December 1982.

Selected compound from the group of anti-androgen compounds disclosed in the DE-OS 31 21 152 are:
(XII) 6-chloro-17-hydroxy-1α,2α-methylenepregna-4,6-diene-3,20-dione;
(XIII) 6-chloro-17-hydroxypregna-4,6-diene-3,20-dione;
(XIV) 6-chloro-17-hydroxypregna-1,4,6-triene-3,20-dione;
(XV) 6-chloro-3,17-dihydroxy-1α,2α-methylenepregna-4,6-diene-20-one;
(XVI) 6-chloro-3-methoxy-17-hydroxy-1α,2α-methylenepregna-4,6-diene-20-one;
(XVII) 6-fluoro-17-hydroxy-1α,2α-methylenepregna-4,6-diene-3,20-dione;
(XVIII) 17-hydroxy-1α,2α-methylenepregna-4,6-diene-3,20-dione;
(XIX) 4,6-dichloro-17-hydroxy-1α,2α-methylenepregna-4,6-diene-3,20-dione.

Preferred are the compounds:
6-chloro-17-hydroxy-1α,2α-methylenepregna-4,6-diene-3,20-dione acetate = cyproterone acetate and
6-chloro-17-hydroxypregna-4,6-diene-3,20-dione acetate (chlormadione acetate)

Selected compound from the group of anti-androgen compounds also disclosed in the DE-OS 31 21 152 are:
(XX) 6-chloro-17αβ-acetoxy-17aα-methyl-1α,2α-methylene-D-homo-4,6-androstadiene-3,17-done;
(XX) 6-chloro-17α-acetoxy-17β-methyl-1α,2α-methylene-D-homo-4,6-androstadiene-3,17a-dione;
(XXI) 2-methyl-N-[4-nitro-3-(triluoromethyl)phenyl]propionamide = flutamide;
(XXII) 2-hydroxy-2-methyl-N-[4-nitro-3-(trifluoromethyl)phenyl]propionamide and
(XXIII) 2-methyl-4-[4-nitro-3-(trifluoromethyl)phenyl]-5,6-dihydro-2H-1,2,4-oxadiazin-3-one.

The preferred anti-oestrogen compound is tamoxifen, the preferred anti-progesterone is onapristone.

### Utility

The combinations of the invention exhibit pharmacological activity and may, therefore, be useful as pharmaceuticals. They can be used as an active therapeutic agent.

In particular, the combinations of the invention show activity in therapy against tumor cell lines. The combinations are active in Balb/c nude mice which have been implanted with tumor cells. The test method and results are described in the Examples 4, 5 and 6.

The combination of TAb 250 and tamoxifen show a significant inhibition of tumor growth at concentration from 0.1 to 10 mg tamoxifen and 100 to 3000 µg of monoclonal antibody using the test method in Balb/c nude mice. Preferred are concentration from 0.1 to 5 mg tamoxifen and 100 to 1000 µg of monoclonal antibody The combinations of the invention are, therefore, useful for the treatment of cancer, especially in using the combination to suppress or inhibit tumors that overexpress the c-erb B-2 receptor.

As the compounds of the combination can be administered by different routes, the invention includes a combination comprising an anti-hormonal compound or compounds and a binding molecule or molecules of the invention as a combined preparation for simultaneous, separate or sequential use. Further the combination comprising an anti-hormonal compound or compounds and a binding molecule or molecules of the invention may be administered as a combined preparation for simultaneous, separate or sequential use, wherein the combination is an active therapeutic agent against tumors.

The invention provides
a) the use of a combination of the invention for the manufacture of a medicament for a therapeutic application for treating cancer, wherein the anti-hormonal compound(s) and the binding molecule(s) of the invention as a combined preparation are simultaneously, separately or sequentially used;
b) a method of treatment of cancer which comprises administering of a cancer suppressing effective amount of a combination of the invention to a patient in need of such treatment, wherein the anti-hormonal compound(s) and the binding molecule(s) of the invention as a combined preparation are simultaneously, separately or sequentially used;
c) a pharmaceutical combination for treatment of cancer which comprises a combination of the invention and a pharmaceutically acceptable diluent or carrier.

Further the invention includes a kit comprising a pharmaceutically active combination of the invention wherein the anti-hormonal compound(s) and the binding molecule(s) of the invention as a combined preparation are simultaneously, separately or sequentially used.

For this indication the appropriated dosage will, of course, vary depending upon, for example, the compounds of the inventions, the host, the mode of administration and the nature and severity of the condition being treated. However, in general, satisfactory results in animals or human beings are indicated to be obtained at daily dosages from about (mAb) 0.01 to about 50 mg/kg body weight and (anti-hormonal c.) from about 0.01 to about 10 mg/kg body weight.
Preferred are daily dosages (mAb) from 0.1 to 10 mg/kg body weight and (anti-hormonal compound) from 0,1 to 10 mg/kg body weight.

The compounds of the invention may be administered by any conventional route, in particular enterally, orally, e.g. in the form of tablets or capsules, or parenterally, e.g. in the form of injectable solutions or suspensions.

The combination of BACh 250 and tamoxifen is the preferred one.

### Process of manufacturing

The invention comprises also a process of manufacturing a combination wherein the anti-hormonal compound is chemically synthesised and
wherein the binding molecule is produced
by immunising an animal with c-erb B-2 protein,
by isolation of B-cells,
by fusing of the B-cells with myeloma cells and selecting positive cells,
by isolating the antibody from the supernatant and in case of need forming fragments of the antibody.

Further the invention comprises a process of manufacturing an inventive combination
wherein the anti-hormonal compound is chemically synthesised and
wherein the binding molecule whose structure is detectable according to the above mentioned process is synthetically or recombinantly produced.

### Description of the Figures

- Figure 1: shows the synergistic effect of a c-erb B-2 antibody (TAb 250) in combination with tamoxifen on MDA-361 cells in athymic (nu/nu) mice.
- Figure 2: shows the synergistic effect of a c-erb B-2 antibody (TAb 250) in combination with tamoxifen on ZR-75-1 cells in athymic (nu/nu) mice.
- Figure 3: shows the synergistic effect on a c-erb B-2 antibody (TAb 250) in combination with the anti-progesterone onapristone on MDA-361 cells in athymic (nu/nu) mice.

### Examples

### Example 1:

### Preparation of c-erb B-2 monoclonal Antibodies

Balb/c mice are immunised intraperitoneally and subcutaneously with 2 · 10⁶ to 2 · 10⁷ NIH3T3 (murine fibroblast cell line obtained from Dr. S. Aaronson, National Institutes of Health) cells transfected with the human c-erb B-2 oncogene, NIH3T3ₜ, (Aaronson S. et al. (1987) Science **237:** 178 - 182) or with a similar number of SKBR3 cells emulsified 1:1 volume/volume in complete Freud's adjuvant. Sera is collected every two weeks and tested for reactivity in an ELISA (enzyme-linked immunosorbent assay) assay against formalin fixed NIH3T3 or NIH3T3ₜ cells. Animals with positive titers are boosted intraperitoneally or intravenously with cells in PBS (phosphate buffered saline), and animals are sacrificed 4 days later for fusion. Spleen cells are fused with P-X63Ag8.653 myeloma cells at a ration of 1:1 to 7.5:1 with PEG 4000 as described by the procedure of Köhler and Milstein ((1975) Nature, **256:** 495 - 497). Fused cells are gently washed and plated in 96-well plates at 1 to 4 · 10⁶ cells / ml in RPMI 1640 medium. Wells are fed with HAT medium 24 hours after the fusion and then every 3 days for 2 to 3 weeks. When colony formation is visible, after 10 to 14 days, the supernatants are tested for reactivity in the ELISA assay. Prospective clones demonstrating good growth are expanded into 24-well plates and re-screeened 7 to 10 days later. Positive wells are then assayed for external domain reactivity against live NIH3T3 and NIH3T3ₜ cells by flow sorting analysis. (For description of the methods see US-Patents No. 3,376,110; 4,016,043 and Harlow and Lane, Antibodies: A Laboratory Manual, Cold Spring Harbor Publications N.Y. (1988)) Clones which are positive both by ELISA assay and flow sorting analysis are recloned either by limiting dilution of by single cell deposition using a flow cytometer. Cells are diluted and deposited into 96-well plates in the presence or absence of spleen feeder cells. Wells demonstrating growth are re-tested by ELISA and recloned an additional three times. Supernatants from hybridoma clones are tested for isotype and subisotype, reactivity to surface expressed gp 185 protein (c-erb B-2) on NIH3T3ₜ cells by flow sorting analysis, and immunoprecipitation of a labeled gp 185 protein from transfected cells. Positive hybridomas are grown in tissue culture and injected into pristane-primed Balb/c mice, Balb/c nude mice or IRCF₁ mice for ascites production. Monoclonal antibodies are purified from ascites fluid by HPLC using a Bakerbond ABx column. Purified monoclonal antibodies (referred to by TAb number) are dialysed against PBS and stored at -20 °C. All purified antibodies are tested for isotype, subisotype, and contamination isotypes by radial immunodiffusion. Cell surface staining of gp 185 expressing cell lines is detected and quantified by flow sorting analysis, ELISA reactivety against purified extracellular domain, ELISA assay

**TABLE 1**

| Characteristics of monoclonal Antibodies recognising the external Domain of c-erb B-2 | | | | | | | |
|---|---|---|---|---|---|---|---|
| TAb¹ | FACS² Binding | ELISA³ Titer(ng/ml) | Subisotype | p185 | p170 | External Domain | Western⁴ Blot |
| 250 | + | 20 | IgG₁ | + | - | + | - |
| 251 | + | 1 | IgG₁ | + | - | + | - |
| 252 | + | 20 | IgG₁ | + | - | + | - |
| 253 | + | 47 | IgG₁ | + | - | + | - |
| 254 | + | 10 | IgG₁ | + | - | + | - |
| 255 | + | 16 | IgG₁ | + | - | +/- | - |
| 256 | + | 25 | IgG₁ | + | - | + | - |
| 257 | + | 11 | IgG₁ | + | - | + | - |
| 258 | + | 4 | IgG₁ | + | - | + | - |
| 259 | + | 20 | IgG₁ | + | - | + | - |
| 260 | + | 10 | IgG₂ₐ | + | - | + | - |
| 261 | + | 10 | IgG_{2b} | + | - | +/- | - |
| 262 | + | 27 | IgG₁ | + | - | + | - |
| 263 | + | 100 | IgG_{2b} | + | - | + | - |
| 264 | + | 41 | IgG₁ | + | - | + | - |
| 265 | + | 51 | IgG₁ | + | - | + | - |
| ND - Not determined | | | | | | | |

| | | | | | | | |
|---|---|---|---|---|---|---|---|
| ¹ Mice are immunised with NIH3T3ₜ except TAb 259 (SKBR-2 cells) | | | | | | | |
| ² FACS - Fluorescence Activated Cell Sorter Analysis | | | | | | | |
| ³ ELISA - ELISA titer is calculated at 30% of maximal binding to NIH3T3ₜ | | | | | | | |
| ⁴ Western blot is carried our under reducing conditions | | | | | | | |

against transfected and untransfected NIH3T3 cells, and radio-immunoprecipitation of p185 from labelled c-erb B-2 expressing cell lines. The antibodies do not cross-react with the closely related EGF-receptor protein as shown by the failure to precipitate a radiolabelled 170 kDalton protein from radiolabelled A-431 cells, and they are analysed by SDS-Page and gel densitometry (all purified proteins are >90% immunoglobulin). A summary of the monoclonal antibodies and the reactivity of these antibodies is outlined in TABLE 1.

### Example 2:

### Inhibition of Breast Tumor Cell Growth

The MTT assay can be used to assess cellular proliferation as a function of mitochondrial activity. (See MOSMANN, T (1983) J Immunol Methods **65:** 55-63) The assay is used to examine effects of the c-erb B-2 monoclonal antibodies and is carried out as follows:

SKBR3 (human breast cancer cell line expressing the c-erb B-2 protein, which originated as a human metastatic pleural effusion obtained from the ATCC catalogue #HTB30) cells are seeded in 96-well microtiter plates (1x10⁴ cells/well), and 24 hours later, dilutions of TAb 252 or a non-specific IgG1 isotype control antibody are added. Plates are incubated for an additional 72 hours. MTT is added for 4 hours and the crystals are dissolved with isopropanol/0.04 N HCL/0.3% SDS. It is found that growth inhibition is restricted to cells expressing the c-erb B-2 protein. TABLE 2 shows the response of SKBR3 cells in contrast to two other breast cell lines that do not express c-erb B-2. Neither HBL 100 (breast cell line derived from human milk gland obtained from the ATCC, catalogue #HTB124) nor MDA468 (breast cancer cell line which originated as a metastatic pleural effusion and contains amplified EGFr obtained from the ATCC, catalogue #HTB132, which overexpresses the EGF receptor) is inhibited by the TAb 252.

**TABLE 2**

| Anti-Proliferative Effect of Anti-c-erb B-2 Antibodies | | | |
|---|---|---|---|
| Antibody | Conc.(µg/ml) | Cell Type | % of Control |
| TAb 252 | 0.1 | HBL100 | 88 |
| | 1.0 | | 96 |
| | 10 | | 95 |
| | 100 | | 108 |
| TAb 252 | 0.1 | MDA468 | 96 |
| | 1.0 | | 95 |
| | 10 | | 94 |
| | 100 | | 90 |
| TAb 252 | 0.1 | SKBR3 | 74 |
| | 1.0 | | 66 |
| | 10 | | 64 |
| | 100 | | 57 |
| TAb 250 | 0.1 | SKBR3 | 100 |
| | 1.0 | | 92 |
| | 10 | | 64 |
| | 100 | | 49 |
| IgG control | 0.1 | SKBR3 | 100 |
| | 1.0 | | 99 |
| | 10 | | 100 |
| | 100 | | 103 |

The effects of TAb 250, another anti-c-erb B-2 monoclonal antibody, on the growth of SKBR3 cells is also shown in TABLE 2. TAb 250 inhibited proliferation up to 50% at a concentration of 100 µg/ml.

Several other monoclonals made against c-erb B-2 are also tested for growth inhibition (TABLE 3). TAbs 250, 252, 253 and 256 all inhibit proliferation to approximately 50 % of control at 100 µg monoclonal antibodies / ml and 75 to 90 % of control at 3.1 µg monoclonal antibodies / ml.

**TABLE 3**

| Effects of c-erb B-2 monoclonal Antibodies on Proliferation of SKBR3 Cells *in vitro* | | | |
|---|---|---|---|
| No. | Antibody | 100 µg/ml | 3.1 µg/ml |
| 1 | IgG1 control | 97¹ | 95 |
| 2 | 250 | 55 | 76 |
| 3 | 250.05² | 53 | 75 |
| 4 | 251 | 72 | 80 |
| 5 | 252 | 55 | 78 |
| 6 | 253 | 44 | 82 |
| 7 | 254 | 69 | 77 |
| 8 | 255 | 63 | 78 |
| 9 | 256 | 50 | 76 |
| 10 | 257 | 68 | 82 |
| 11 | 258 | 75 | 82 |
| 12 | 259 | 73 | 85 |
| 13 | 260 | 73 | 91 |
| 14 | 261 | 88 | 95 |
| 15 | 262 | 74 | 88 |
| 16 | 263 | 70 | 86 |
| 17 | 264 | 69 | 90 |
| 18 | 265 | 62 | 80 |

| | | | |
|---|---|---|---|
| ¹ Numbers are expressed as percent of untreated control cells | | | |
| ² This represents TAb 250 purified from a different batch | | | |

### Eample 3:

### Inhibition of Ovarian Tumor Cell Growth

To determine the effect of various antibodies reactive with the c-erb B-2 protein on tumor cell proliferation, SKOV3 (human ovarian cancer cell line expressing the c-erb B-2 protein, which originated as a metastatic ascitic effusion obtained from the ATCC, catalogue #HTB77) cells are seeded in growth medium into 24-well dishes at 10,000 cells / well. After 24 hours at 37 °C, monoclonal antibodies (TAbs 250, 251, 257, 261, 263) or purified Fab or F(ab')₂ fragments of TAb 250 are added to yield a final assay concentration of 10 µg/ml. Ten days after the addition of antibodies, the cells are removed with trypsin and quantified using a Culture Counter. In addition, representative cell samples are stained with propidium iodide and analysed using a FACS Scan (Becton-Dickinson, Mountain view, CA) to determine the percentage of viable cells in each treatment group. Thus, each value in TABLE 4 represents the mean of triplicates and is expressed as percentage of the viable cell number compared to untreated control wells. Neither the number nor the viability of cells treated with TAbs 261, 263 or the Fab fragment of TAb 250 is different from untreated controls. However, after 10 days in the presence of TAbs 250, 251 or 257 the proliferation of SKOV3 cells is suppressed to 69%, 64 % or 60 % of control cell number. Treatment with F(ab')₂ fragments of TAb 250 reduced cell growth to 76% of control levels. While control cells are assessed to be >98% viable, cells treated with these antibodies demonstrated a small but significant loss of viability (from 84 to 89%).

**TABLE 4**

| Effect of anti-c-erb B-2 Antibodies on ovarian Tumor Cell Growth | |
|---|---|
| Antibody | % of control |
| TAb 250 | 69 |
| TAb 251 | 64 |
| TAb 257 | 60 |
| TAb 261 | 89 |
| TAb 263 | 88 |
| TAb 250 Fab | 101 |
| TAb 250 F(ab)₂ | 77 |

### Example 4:

### Effect of c-erb B-2 monoclonal Antibody and Tamoxifen in Combination on human Tumor Xenografts (MDA 361 cells)

Slow release (60 days) oestrogen pellets (0.72 mg/pellet, Innovative Research, Inc.) or placebo pellets (Innovative Research) are implanted into 80 Balb/c nude mice. After one day, the mice are implanted with MDA-MB-361 (human breast cancer cell line) expressing the c-erb B-2 protein, which originated as a metastatic tumor to the brain obtained from the ATCC, catalogue #HTB27) cells. The tumor cells are grown in roller bottles, harvested and diluted with PBS to 1.1x10⁷ cells per 100 µl. On day seven following tumor cell implantation, the mice are randomly subdivided into ten groups of eight mice each. The mice are implanted with slow release pellets containing either placebo or tamoxifen (2.5 or 5.0 mg). On the same day the mice are also treated intraperitoneally with TAb 250 (500 or 1000 µg) or non-specific IgG1 isotype control antibody. Treatment of the various groups is as follows:
- Group 1 -: placebo pellet implant and 1,000 µg IgG1 isotype control;
- Group 2 -: no tamoxifen and 500 µg TAb 250
- Group 3 -: no tamoxifen and 1000 µg TAb 250;
- Group 4 -: 2.5 mg tamoxifen and no antibody;
- Group 5 -: 5.0 mg tamoxifen and no antibody;
- Group 6 -: 5.0 mg tamoxifen and 1,000 µg IgG1 isotype control;
- Group 7 -: 2.5 mg tamoxifen and 500 µg TAb 250;
- Group 8 -: 2.5 mg tamoxifen and 1000 µg TAb 250;
- Group 9 -: 5.0 mg tamoxifen and 500 µg TAb 250;
- Group 10 -: 5.0 mg tamoxifen and 1000 µg TAb 250.

Additional intraperitoneal treatments are given on days 9, 11, 14, 16, 18, 21, 23, and 25 following tumor implantation.

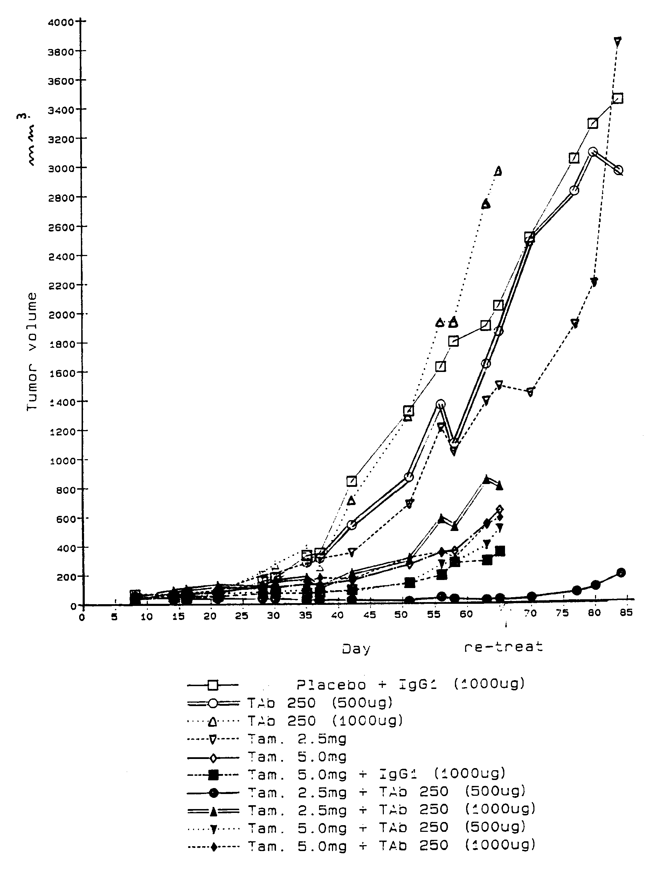

The results of this experiment are shown in Figure 1. Significant inhibition of tumor growth is observed in the mice treated with the combination of TAb 250 and tamoxifen, especially at the combined dosages of 2.5 mg tamoxifen and 500 µg of TAb 250. Four of eight mice had no measurable tumour at day 50 and seven of eight mice had either no measurable tumor or tumors too small to measure at day 84.

### Example 5:

### Effect of a c-erb B-2 monoclonal Antibody and Tamoxifen in Combination of human Tumor Xenografts (ZR-75-1 cells)

A second experiment is conducted essentially as described above using ZR-75-1 cells (human breast cancer cell line, ATCC #CRL 1500). Slow release oestrogen pellets or placebo pellets are implanted into 40 Balb/c nude mice. One day after pellet implantation, the oestrogen implanted mice are injected sub-cutaneously with 100 µl of ZR-75-1 tumor cell suspension prepared as follows. ZR-75-1 tumors are excised from untreated passage mice at about 3400 mm³, finely minced and suspended in PBS. Six days following tumor cell implantation, the mice are randomly subdivided into five groups of eight mice each. The mice are implanted with slow release pellets containing either placebo or 2.5 mg tamoxifen. On day 12 following tumor cell implantation the mice are treated intraperitoneally with either 500 µg TAb 250 or an equivalent volume of PBS. Treatment of the various groups is as follows.
- Group 1 -: placebo pellets and PBS;
- Group 2-: 2.5 mg tamoxifen and no antibody;
- Group 3 -: 500 µg TAb 250;
- Group 4 -: 2.5 mg tamoxifen and PBS;
- Group 5 -: 2.5 mg tamoxifen and 500 µg TAb 250.

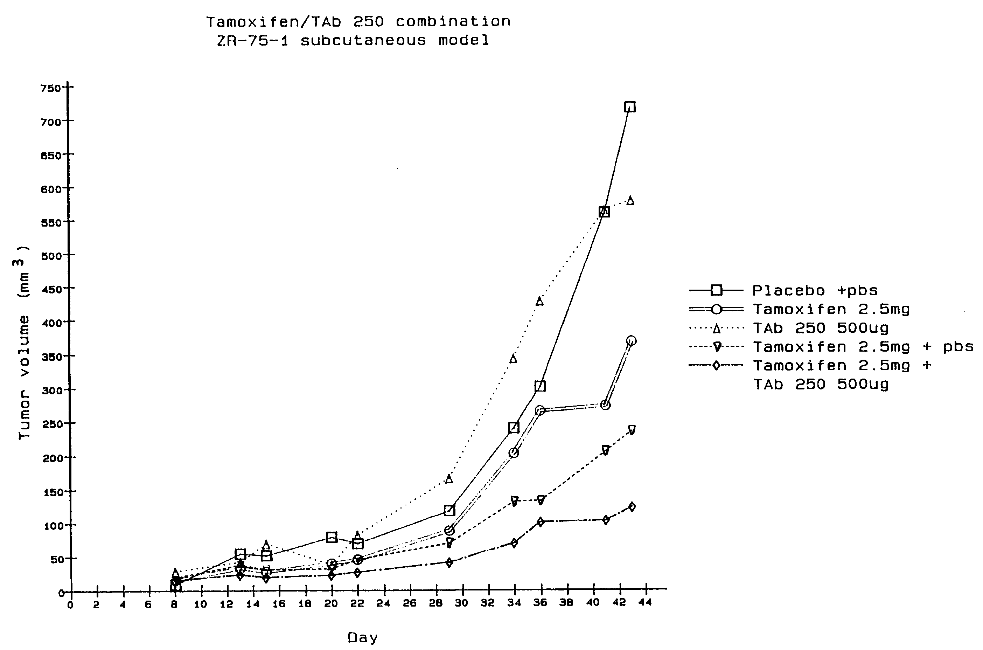

Additional intraperitoneal treatments are given on days 14, 16, 19, 21, 23, 28, and 30 following tumor cell implant. The results of this experiment are shown in Figure 2. Clear inhibition of tumor growth is observed in the mice treated with the TAb 250 and tamoxifen combination in contrast to other treatment regimens.

### Example 6:

### Effect of a c-erb B-2 monoclonal Antibody and Onapristone in Combination on human Tumor Xenografts

Slow release oestrogen or placebo pellets are implanted into 48 Balb/c nude mice. After one day, the mice are implanted subcutaneously with approximately 1.3x10⁶ cells in 100 µl of PBS prepared from cells grown in tissue culture flasks (see Example 4). The mice are randomly subdivided into six treatment groups of 8 mice each. On day 4 following cell implantation, the groups are treated as follows:
- Group 1 -: 10% benzyl benzoate in castor oil (control);
- Group 2-: 500 µg TAb 250;
- Group 3 -: 20 µg onapristone;
- Group 4 -: 20 µg onapristone and 500 µg TAb 250;

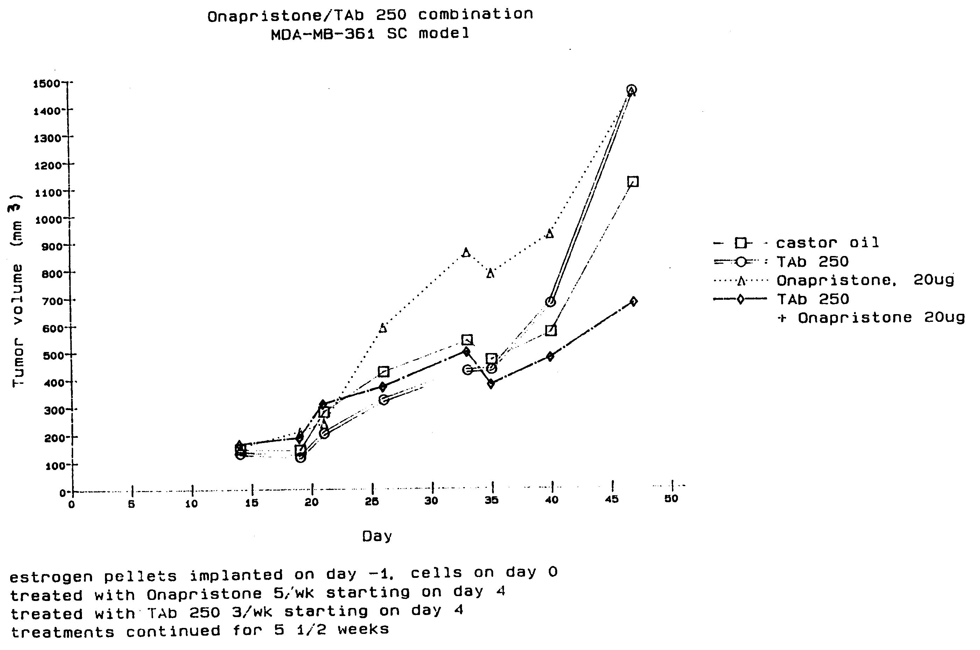

Onapristone is administered subcutaneously in a solution of 10% benzyl benzoate/90% castor oil. TAb 250 is administered via intraperitoneal injection. Treatments (50 µl total volume) of onapristone are given daily for 5 consecutive days by subcutaneous injection. The mice are then rested for two days, and another cycle of 5 days subcutaneous injections is given. There are 5.5 such cycles for a total of 28 injections per mouse. Treatments of TAb 250 are given 3 times per week for 5.5 weeks for a total of 17 treatments. The results of this experiment are shown in Figure 3. Clear inhibition of tumor growth is observed in mice treated with TAb 250 and onapristone in combination.

## Claims

1. A combination comprising
a) at least one anti-hormonal compound taken from the groups comprising anti-oestrogen, anti-progesterone and anti-androgoen compounds and
b) a binding molecule or binding molecules specifically binding the protein encoded by the c-erb B-2 oncogene,
wherein the binding molecule or molecules induce inhibition of tumor cell proliferation.

2. A combination according to claim 1 wherein the binding molecule or molecules are an antibody or fragments thereof.

3. A combination according to claim 1 wherein the anti-hormonal compound is tamoxifen or derivatives thereof.

4. A combination according to claim 1 wherein the anti-hormonal compound is onapristone or derivatives thereof.

5. A combination according to claim 1 wherein the anti-hormonal compound is dihydrospirorenone.

6. A combination according to claim 1 wherein the anti-hormonal compounds are a mixture of two or three compounds taken from the groups comprising anti-oestrogen, anti-progesterone and anti-androgen compounds.

7. A combination according to any one of claims 1 to 6 as an active therapeutic agent.

8. A combination comprising an anti-hormonal compound or compounds and a binding molecule or molecules according to any one of claims 1 to 6 as a combined preparation for simultaneous, separate or sequential use.

9. A combination comprising an anti-hormonal compound or compounds and a binding molecule or molecules according to any one of claims 1 to 6 as a combined preparation for simultaneous, separate or sequential use, wherein the combination is an active therapeutic agent against tumors.

10. Use of a composition according to any one of claims 1 to 6 for the manufacture of a medicament for a therapeutic application for treating cancer, wherein the anti-hormonal compound(s) and the binding molecule(s) according to any one of claims 1 to 6 as a combined preparation are simultaneously, separately or sequentially used.

11. A pharmaceutical composition comprising the combination of any one of claims 1 to 6 together with at least one pharmaceutically acceptable diluent or carrier.

12. A kit comprising a pharmaceutically active combination according to any one of the claims 1 to 6 wherein the anti-hormonal compound(s) and the binding molecule(s) according to any one of claims 1 to 6 as a combined preparation are simultaneously, separately or sequentially used.

13. A process of manufacturing a combination according to any one of claims 1 to 6
wherein the anti-hormonal compound is chemically synthesised and
wherein the binding molecule is produced
by immunising an animal with the c-erb B-2 protein,
by isolation of B-cells,
by fusing of the B-cells with hybridoma cells and selecting positive cells,
by isolating the antibody from the supernatant and in case of need forming fragments of the antibody.

14. A process of manufacturing a combination according to any one of claim 1 to 6,
wherein the anti-hormonal compound is chemically synthesised and
wherein the binding molecule which structure is detectable according to the process of claim 13 is synthetically or recombinantly produced.

## Patentansprüche

1. Kombination umfassend
a) mindestens eine antihormonell wirksame Verbindung aus den Gruppen umfassend Antiöstrogen-, Antiprogesteron- und Antiandrogenverbindungen sowie
b) ein Bindungsmolekül bzw. Bindungsmoleküle, das bzw. die spezifisch an das vom Oncogen c-erb B-2 codierte Protein bindet bzw. binden,
wobei das Bindungsmolekül bzw. die Bindungsmoleküle die Hemmung der Tumorzellproliferation induzieren.

2. Kombination nach Anspruch 1, wobei es sich bei dem Bindungsmolekül bzw. den Bindungsmolekülen um einen Antikörper oder dessen Fragmente handelt.

3. Kombination nach Anspruch 1, wobei es sich bei der antihormonell wirksamen Verbindung um Tamoxifen oder dessen Derivate handelt.

4. Kombination nach Anspruch 1, wobei es sich bei der antihormonell wirksamen Verbindung um Onapriston oder dessen Derivate handelt.

5. Anspruch 1, wobei es sich bei der antihormonell wirksamen Verbindung um Dihydrospirorenon handelt.

6. Kombination nach Anspruch 1, wobei es sich bei den antihormonell wirksamen Verbindungen um eine Mischung aus zwei oder drei Verbindungen aus den Gruppen umfassend Antiöstrogen-, Antiprogesteron- und Antiandrogenverbindungen handelt.

7. Kombination nach einem der Ansprüche 1 bis 6 als therapeutischer Wirkstoff.

8. Kombination umfassend eine antihormonell wirksame Verbindung bzw. antihormonell wirksame Verbindungen und ein Bindungsmolekül bzw. Bindungsmoleküle nach einem der Ansprüche 1 bis als Kombinationspräparat für die gleichzeitige, getrennte oder aufeinanderfolgende Verwendung.

9. Kombination umfassend eine antihormonell wirksame Verbindung bzw. antihormonell wirksame Verbindungen und ein Bindungsmolekül bzw. Bindungsmoleküle nach einem der Ansprüche 1 bis 6 als Kombinationspräparat für die gleichzeitige, getrennte oder aufeinanderfolgende Verwendung, wobei es sich bei der Kombination um einen therapeutischen Wirkstoff gegen Tumore handelt.

10. Verwendung einer Zusammensetzung nach einem der Ansprüche 1 bis 6 für die Herstellung eines Arzneimittels zur Krebstherapie, wobei die antihormonell wirksame(n) Verbindung(en) und das bzw. die Bindungsmolekül(e) nach einem der Ansprüche 1 bis 6 als Kombinationspräparat gleichzeitig, getrennt oder nacheinander verwendet werden.

11. Pharmazeutische Zusammensetzung umfassend die Kombination nach einem der Ansprüche 1 bis 6 gemeinsam mit mindestens einem pharmazeutisch unbedenklichen Streckmittel oder Träger.

12. Kit umfassend eine pharmazeutisch wirksame Kombination nach einem der Ansprüche 1 bis 6, wobei die antihormonell wirksame(n) Verbindung(en) und das bzw. die Bindungsmolekül(e) nach einem der Ansprüche 1 bis 6 als Kombinationspräparat gleichzeitig, getrennt oder nacheinander verwendet werden.

13. Verfahren zur Herstellung einer Kombination nach einem der Ansprüche 1 bis 6,
bei der die antihormonell wirksame Verbindung chemisch synthetisiert wird und
bei der das Bindungsmolekül dadurch produziert wird, daß man
ein Tier mit dem Protein c-erb, B-2 immunisiert, B-Zellen isoliert,
die B-Zellen mit Hybridomzellen fusioniert und positive Zellen selektiert,
den Antikörper aus dem Überstand isoliert und gegebenenfalls Fragmente des Antikörpers bildet.

14. Verfahren zur Herstellung einer Kombination nach einem der Ansprüche 1 bis 6,
bei der die antihormonell wirksame Verbindung chemisch synthetisiert wird
und bei der das Bindungsmoleküle, welche Struktur nach dem Verfahren nach Anspruch 13 nachweisbar ist, auf synthetischem oder rekombinantem Weg produziert wird.

## Revendications

1. Combinaison comportant
a) au moins un composé antihormonal provenant des groupes comprenant des composés antioestrogène, antiprogestérone et antiandrogène et
b) une molécule de liaison ou des molécules de liaison fixant spécifiquement la protéine codée par l'oncogène c-erb B-2,
où la ou les molécules de liaison induisent une inhibition de la prolifération des cellules tumorales.

2. Combinaison suivant la revendication 1, où la ou les molécules de liaison sont un anticorps ou des fragments de celui-ci.

3. Combinaison suivant la revendication 1, où le composé antihormonal est le tamoxifène ou des dérivés de celui-ci.

4. Combinaison suivant la revendication 1, où le composé antihormonal est l'onapristone ou des dérivés de celui-ci.

5. Combinaison suivant la revendication 1, où le composé antihormonal est le dihydrospirorénone.

6. Combinaison suivant la revendication 1, où les composés antihormonaux sont un mélange de deux ou trois composés provenant des groupes comprenant des composés antioestrogène, antiprogestérone et antiandrogène.

7. Combinaison suivant l'une quelconque des revendications 1 à 6, en tant qu'agent thérapeutique actif.

8. Combinaison comportant un ou des composés antihormonaux et une' ou des molécules de liaison suivant l'une quelconque des revendications 1 à 6, en tant que préparation combinée destinée à un usage simultané, séparé ou séquentiel.

9. Combinaison comportant un ou des composés antihormonaux et une ou des molécules de liaison suivant l'une quelconque des revendications 1 à 6, en tant que préparation combinée destinée à un usage simultané, séparé ou séquentiel, où la combinaison est un agent thérapeutique actif contre les tumeurs.

10. Utilisation d'une composition suivant l'une quelconque des revendications 1 à 6, pour la fabrication d'un médicament destiné à un usage thérapeutique dans le traitement du cancer, où le (les) composé(s) antihormonal(aux) et la (les) molécule(s) de liaison suivant l'une quelconque des revendications 1 à 6, en tant que préparation combinée, sont utilisés simultanément, séparément ou séquentiellement.

11. Composition pharmaceutique comportant la combinaison de l'une quelconque des revendications 1 à 6, conjointement avec au moins un diluant ou un excipient acceptable sur le plan pharmaceutique.

12. Kit comportant une combinaison active sur le plan pharmaceutique suivant l'une quelconque des revendications 1 à 6, où le (les) composé(s) antihormonal(aux) et la les molécule(s) de liaison suivant l'une quelconque des revendications 1 à 6, en tant que préparation combinée, sont utilisés simultanément, séparément ou séquentiellement.

13. Procédé de fabrication d'une combinaison suivant l'une quelconque des revendications 1 à 6,
où le composé antihormonal est synthétisé chimiquement et
où la molécule de liaison est produite
en immunisant un animal avec la protéine c-erb B-2,
en isolant des cellules B,
en fusionnant les cellules B avec des hybridomes et en sélectionnant des cellules positives,
en isolant l'anticorps du surnageant et, si nécessaire, en formant des fragments de l'anticorps.

14. Procédé de fabrication d'une combinaison suivant l'une quelconque des revendications 1 à 6,
où le composé antihormonal est synthétisé chimiquement et
où la molécule de liaison, dont la structure peut être détectée conformément au procédé de la revendication 13, est produite par synthèse ou par recombinaison.
